# EUROPEAN PATENT SPECIFICATION

(11) **EP 2 552 338 B1**
(45) Date of publication and mention of the grant of the patent: **18.08.2021**
(21) Application number: 11763356.0
(22) Date of filing: 30.03.2011
(51) Int. Cl.: A61B 46/00

(54) **SURGICAL DRAPE HAVING TEARABLE SHEET**
OP-ABDECKTUCH MIT AUFREISSBARER FOLIE
CHAMPS OPÉRATOIRES AYANT UNE FEUILLE POUVANT ÊTRE DÉCHIRÉE

(30) Priority: 31.03.2010 US 319738 P
(43) Date of publication of application: 06.02.2013
(73) Proprietor: Allegiance Corporation, McGaw Park, IL 60085 (US)
(72) Inventor: REYES, Rogelio, Elpaso, TX 79922 (US); ROBARDS, Douglas, Gurnee, IL 60031 (US)
(74) Representative: Prock, Thomas
(86) International application number: PCT/US2011/030468
(87) International publication number: WO 2011/123506

(56) References cited:
- EP-A2- 2 151 211
- WO-A1-99/04721
- GB-A- 2 439 640
- US-A- 3 072 511
- US-A- 4 041 942
- US-A- 5 532 053
- US-A- 5 975 082
- US-A1- 2010 031 966

## Description

### FIELD OF THE INVENTION

The present invention relates generally to surgical devices, and specifically to surgical drapes, for use during surgery.

### BACKGROUND OF THE INVENTION

Surgical drapes are an important consideration in the medical field. It is well known to cover patients undergoing surgery with surgical drapes to create a sterile barrier around the surgical site. Some surgical drapes have fenestrations, or predefined openings, used during the procedures for one of two primary purposes, namely, to give access through the drape to the surgical site or to accommodate a portion of the patient's anatomy. In either case, the fenestration provides an opening in the drape to isolate the surgical site, and thereby create a sterile field between the body portion containing the surgical site and the remainder of the patient's body.

During surgeries, surgeons need to control the placement of surgical equipment at the surgical site, such as catheters, and the like. Once the surgery is completed, surgeons need a simple and safe way to remove surgical drapes away from the surgical site without disturbing the surgical equipment, such as catheters.

U.S. Patent No. 6,405,730 discloses an ophthalmic surgical drape having a fenestration and at least two tear lines extending from the fenestration. Because the tear lines are perforated or scored, the drape contains a series of holes, which is counter to the purpose of using the drape.

U.S. Patent No. 5,975,082 discloses a drape having a fenestration and score lines extending from the fenestration. Because the tear lines are perforated or scored, the drape contains a series of holes, which is counter to the purpose of using the drape.

U.S Patent Application Publication No. 2010/0031966 discloses a pre-cut drape held together by a tape having a scoreline formed by partially severing the tape through its thickness. The tape includes the score line to allow the tape to be torn linearly along its length. However, because the tape include the score line, it is unstable and easily prone to unintentional tearing when the drape is manipulated.

WO 99/04721 describes a tear-away surgical drape. The drape has a fluid absorbent pad attached to the upper surface of an underlying base sheet. The drape has an opening, or fenestration therein through which the operation is to be performed. The absorbent pad has a score line in it which extends from n edge of the pad to the fenestration so that it, like the tear-away material forming the base sheet, may be easily torn away after surgery.

EP 2 151 211 describes a medical drape having a tool-less removal feature and includes a drape material, a drape cut, an adhesive tape strip, and a scoreline. The drape material has a top side, a back side, and at least one exterior edge. The drape cut has a starting point at the exterior edge and extends completely through the thickness of the drape material.

GB 2 439 640 describes a surgical drape for use in tracheostomy procedures that has a main opaque body region and a transparent region arranged to extend over the head of the patient. An incise aperture is formed in the body region close to the transparent head region for providing access to the throat. A perforated tear line may extend laterally from the aperture to the edge of the drape to enable the drape to be torn easily so that it can be removed from around any tubing projecting through the aperture. Preferably the main region is of woven, water-repellent material and the transparent region is polyethylene.

### SUMMARY OF THE INVENTION

There is an unmet need for a surgical drape that is removable from around a surgical site without requiring the use of additional tools, such as scissors. Furthermore, there is an unmet need for a surgical drape that is removable without disturbing the surgical equipment and without implementing perforations or a series of holes in the surgical drape or scores in a securing tape.

The present invention is defined by the appended independent claim 1. Further preferred embodiments are disclosed in the dependent claims. Independent claim 23 further discloses a method of removing the device in claim 1.

The present invention also provides for a method of removing the surgical drape invention from a surgical site without disturbing surgical equipment located at the surgical site.

The above and still other advantages of the invention will be apparent from the detailed description and drawings. What follows are one or more preferred embodiments of the present invention.

### DESCRIPTION OF THE DRAWINGS

FIG. 1 is a front view of a surgical drape;
FIG. 2 is a cross-section view of the drape of FIG. 1 taken along line 2-2;
FIG. 3 is a cross-section view of the drape of FIG. 1 taken along line 3-3;
FIG. 4 is a front view of the drape of FIG. 1 in a torn state;
FIG. 5 is a front view of a second aspect of a surgical drape of the present invention;
FIG. 6 is a cross-section view of the drape of FIG. 5 taken along section line 6-6;
FIG. 7 is a cross-section view of the drape of FIG. 5 taken along section line 7-7; and
FIG. 8 is a front view of the drape of FIG. 5 in a torn state.

### DETAILED DESCRIPTION OF THE INVENTION

The present invention provides for a surgical drape and a method of removing a surgical drape. The present invention provides for a surgical drape comprising a tearable first sheet, the first sheet comprising at least one notch disposed at a periphery of the first sheet, and an imperforate portion extending from the at least one notch to at least one fenestration, wherein tearing the first sheet via the at least one notch creates a linear tear line extending along the imperforate portion from the at least one notch to the at least one fenestration. In a first aspect the at least one fenestration is formed in the first sheet. In a second aspect the surgical drape includes a second sheet having a first and a second surface, wherein the second sheet comprises the at least one fenestration, wherein the at least one fenestration provides access to a surgical site, wherein a cut extends from a periphery of the second sheet to the at least one fenestration in the second sheet, wherein the first sheet is affixed to the first surface of the second sheet and extends along a length of and overlapping the cut, and wherein the at least one notch is disposed at the periphery of the second sheet and is aligned with the cut. The present invention further provides for a method of removing the surgical drape by tearing the first sheet by applying a tearing force at the at least one notch, and removing the drape from a surgical site. The present invention can be used to effectively and simply allow a surgeon to remove a surgical drape from a surgical site without the use of any additional tools or perforations, and without disturbing any surgical equipment present inside the fenestration.

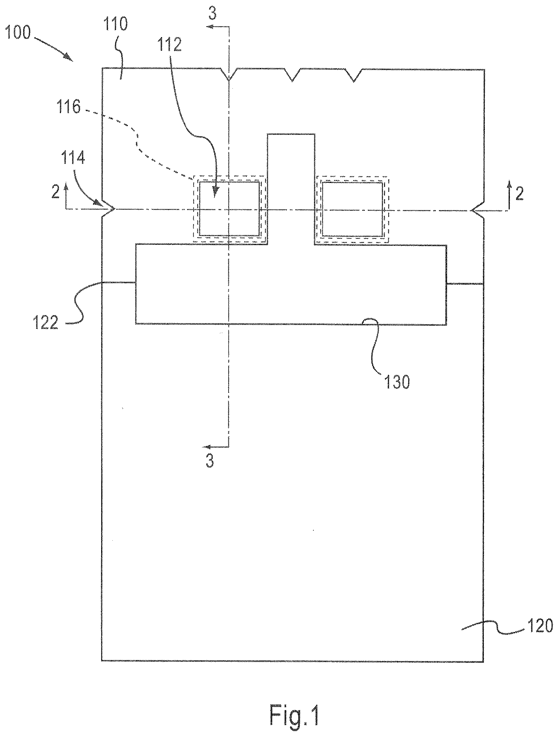

FIGS. 1 to 4 illustrate a first aspect of the surgical drape 100 for use during a surgical procedure. The surgical drape 100 comprises a first sheet 110, an optional second sheet 120 and a reinforcing material 130.

The first sheet 110 is a flexible sheet of material, generally rectangular in a preferred aspect, but can be any suitable shape, provided it is large enough to cover a human body or a portion of the body adjacent the surgical site to assist in creating a sterile field at the surgical site. At least a portion of the first sheet is imperforate. Imperforate means there are no perforated lines, score lines, partial-score lines, noncontinuous cut lines, or any other series of holes, deformations, dimples, depressions, or points of reduced thickness for facilitating a tear line through a material. In a preferred embodiment, the first sheet 110 has at least one opening or fenestration 112, generally disposed in a region towards the center of the drape. A fenestration, however, may be located at any position to allow access to a surgical site. For example a fenestration may be located more towards the center or more towards the periphery of the first sheet. As shown in FIG. 1, in a preferred embodiment, the fenestrations are generally rectangular in shape and provide access to a surgical site located on an arm, such as, above the elbow. In such a surgery, peripherally inserted central catheters may be located within a fenestration. It is within the scope of the invention, however, that the fenestrations may be formed in any suitable shape depending upon the type of surgery. For example, the fenestrations may be oval, circular, triangular, rectangular, or any other suitable shape. Furthermore, in a preferred aspect shown in FIG. 1, there are two fenestrations, however, any number of fenestrations may be included in any orientation as necessary for a particular type of surgery.

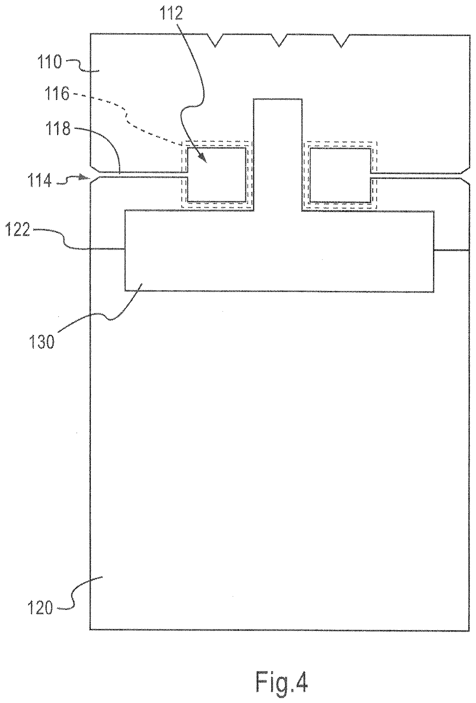

In a preferred aspect, at least a portion of the first sheet 110 is a transparent film comprising of a thermoplastic resin that, when torn, tears in a linear fashion, as best seen in FIG. 4. Furthermore, as shown in FIG. 4, the resin allows tearing in multiple directions. In a preferred embodiment, the resin may comprise poly(ethylene-co-methacrylic acid) polymer, commercially available as Surlyn® from DuPont Company of Wilmington, Delaware. The first sheet may be a single layer or may have multiple layers and the entire first sheet may be formed from the transparent film. In preferred aspects, the first sheet or portion of the first sheet may comprise a three layer laminate, where each of the three layers comprises a resin. It is within the scope of the invention that any known or future developed materials may be used for the resin, as long as a linear tear is produced when the first sheet or portion of the first sheet is torn. Furthermore, in a preferred aspect, the first sheet 110 is about 76 microns (3 mils) thick, however, any thickness is within the scope of the invention as long as a linear tear is produced when the first sheet is torn. For example, the first sheet may be from about 51 microns (2 mils) to about 102 microns (4 mils) thick. Furthermore, while the first sheet is generally transparent to allow a surgeon to see the operative area, the first sheet may also be opaque or semi-opaque if necessary for a particular surgery. The first sheet may also be shaded with any color.

The first sheet 110 further comprises at least one notch 114 located at a periphery of the first sheet. The term notch is meant to include any interruption in the border of the sheet that serves as a starting point for tearing without the aid of a tool, such as a pair of scissors. Thus, the term notch includes, nick, slit, slot, split, slash, cut, divide, and the like. In a preferred aspect a notch 114 is positioned such that the notch directly opposes the fenestration 112. It is within the scope of the invention that any number of notches may be used and in any combination. For example, as shown in FIG. 1, each fenestration has a first notch disposed in a horizontal direction and a second notch disposed in a vertical direction. Any setup of notches, however is within the scope of the invention. For example, each fenestration could have a single notch located in a horizontal direction, each fenestration could have a single notch located in a vertical direction, one fenestration could have a single notch in a horizontal direction while another fenestration has a single notch located in a vertical direction, or one fenestration could have at least one notch associated with it while another fenestration has no notches associated with it. The first sheet may optionally comprise additional notches that do not line up with the fenestrations. For example, as shown in FIGs. 1 and 4, a notch may be disposed at a periphery of the first sheet such that a tear originating at the notch will expose the head of a patient without intersecting with a fenestration.

ln a preferred aspect, the notch may be triangular in shape. As shown in FIG. 1, the triangular notch is oriented such that a point of the notch points directly towards the fenestration 112. A triangular notch having such an orientation allows the surgeon to easily start a tear line at the apex of the triangle notch. Furthermore, a triangular notch prevents curling of the sheet at the edges, which can occur with a vertical or horizontal slit. It is within the scope of the invention, however, that any shaped notch or equivalent thereof can be used. For example, the notch may be a slit or any other shape capable of introducing a linear tear line into the first sheet. Generally, the notch is large enough to allow a surgeon to begin a tear line by applying a tearing force at the notch.

Because the composition of at least a portion of the first sheet only allows for linear tear lines, when the first sheet is spread apart at the notch, as seen in FIG. 4, a tear line 118 forms beginning at the notch and continuously extends perpendicular to the base of the triangle notch or, generally, the periphery of the first sheet on which the notch is located, in a direction of the fenestration 112, until it reaches the fenestration. In an aspect of the present invention, the portion of the first sheet that comprises the resin and produces a linear tear line when torn (i.e., the imperforate portion), extends from the notch to the fenestration. Accordingly, it also within the scope of the invention that portions of the sheet that do not need to be torn may be made of any drape material known in the art. After the tear line is formed between the notch and the fenestration, the surgeon can easily remove the drape without disturbing any surgical equipment present inside of the fenestration. Because the first sheet or portion of the first sheet is tearable in a linear direction, no perforations or score lines need to be formed through the first sheet and no additional equipment, such as scissors is necessary to gain access to the fenestration.

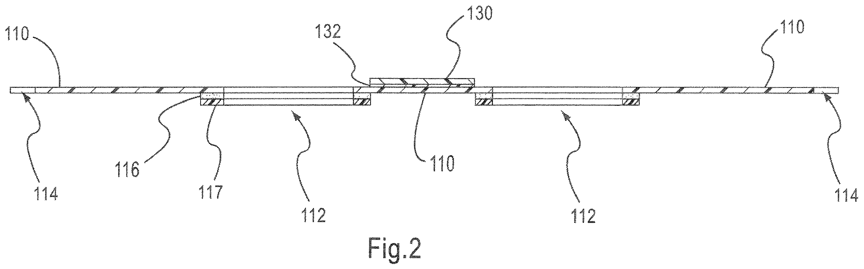

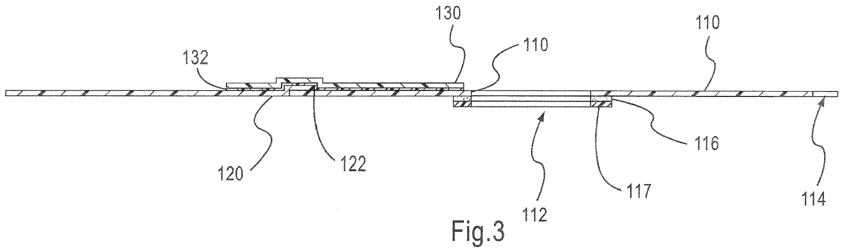

The first sheet optionally further comprises an adhesive disposed on an underside surface of the first sheet, i.e., the side that contacts the patient in use. The adhesive is preferably disposed along the periphery of the fenestration to secure the drape to the patient. The adhesive acts as an anchor for stabilizing the drape and forms a barrier around the fenestrations. In a preferred aspect the adhesive may be an acrylic adhesive. It is within the scope of the invention however that any suitable adhesive may be used, for example, synthetic rubber. As seen in FIGs 2 and 3, the adhesive is applied to the underside surface of first sheet. The adhesive may be applied directly to the first sheet, e.g. with hot melt equipment. Alternatively, the adhesive may be applied via double sided tape. The double sided tape comprises adhesive disposed on both sides of a carrier material. One side of the double sided tape is disposed against the underside surface of the sheet and the other side of the tape is disposed against the patient. The double sided tape may further comprise a removable liner 117 that protects the tape from exposure until the first sheet is ready to be placed on the patient. The amount of the adhesive used will depend on how strong the adhesive must be for a particular surgery. Generally, enough adhesive should be used to securely attach the underside surface of the first sheet to the patient. In an alternate aspect, instead of applying adhesive on the underside surface of the first sheet, a single sided tearable tape may be placed on a top surface of the first sheet. In this aspect, the tape is placed onto the top surface of the first sheet such that a portion of the adhesive side of the tape overlaps a portion of the fenestration. The portion of the adhesive tape that overlaps a portion of the fenestration is freely open to be adhered to the patient and acts to secure the drape to the patient. Before the drape is in use, a removable liner may be placed over the exposed portion of the adhesive tape.

. When the adhesive is applied directly on the underside surface of the first sheet, the type of adhesive should allow the tear line to pass through the adhesive. When double sided tape is applied to the underside surface of the first sheet or when single sided tape is applied to the top surface of the first sheet, the carrier material should be composed of a tearable material, such as a tissue paper. It is within the scope of the invention, however, that any carrier material may be used so long as the the carrier material is strong enough to give body to the adhesive and is tearable. The carrier material need not produce a linear tear when torn along the tear originating from the notch. Because the tape is tearable, no perforations or score lines are needed in the carrier material, and no additional equipment, such as scissors, is needed to gain access to the fenestration.

The drape 100 optionally further comprises a second sheet 120. The second sheet may be a generally rectangular shaped sheet for covering areas remote from the surgical site. For example, the second sheet may be used to cover the parts of the patient not being operated on, surgical equipment nearby the patient, and medical staff. While the second sheet is typically larger that the first sheet, the second sheet may be any suitable size or shape necessary for the particular operation. The second sheet can be made of any of a variety of suitable commercially available medical fabric materials. Such medical fabric materials known in the surgical field include non-woven fabrics. Non-woven fabrics refer to a single web, or an assembly or laminate of multiple webs, formed of individual randomly laid fibers, for example using a spunlaid, thermobonded, spunbonded, meltblown or bonded carded web process. A laminate of non-woven fabrics is one conventional material in the surgical field that could be used for the second sheet. A spunbonded/meltblown/spunbonded (SMS) laminate of polypropylene fibers is one example. Another example of a suitable medical fabric material for second sheet is a combination non-woven fabric and film in which a liquid impervious polymer film is disposed between two non-woven layers. Such a spunbonded/film/spunbonded laminate material is commercially available as Tiburon® from Ahistrom Corporation of Helsinki, Finland.

One end of the second sheet may be attached to an end of the first sheet, such as via an adhesive. Any adhesive suitable for securing the second sheet to the first sheet may be used.

As seen in FIGs. 1 and 3, the drape 100 further comprises a reinforcing material 130 disposed on a surface of the first sheet 110 and a surface of the second sheet 120, preferably on the surface opposite the underside surface where the optional adhesive may be located. The reinforcing material may comprise the same types of material as the second sheet. However, the basis weight of the reinforcing material may be greater than the basis weight of the second sheet.

A function of the reinforcing material is to absorb fluids produced during the course of the surgical procedure. Thus, the reinforcing material may comprise an absorbent material. The material may be made absorbent through any suitable treatment, e.g. by surfactant surface treatment. Alternatively, the reinforcing material may be treated to repel fluids.

The reinforcing material is shaped and placed to surround as much of the fenestration as possible without intersecting the path of the tear line originating from the notch. For example, in a preferred aspect, shown in FIG. 1, the reinforcing material 130 is a t-shape so that it may fit between and surround two sides of the fenestrations. Alternative shapes may be utilized to surround three sides of the fenestrations. Furthermore, in a preferred aspect, the reinforcing material may comprise a film layer 132. The film layer may be used to secure the reinforcing material to a surface of the first sheet and a surface of the second sheet, e.g. with hot melt equipment. In a preferred aspect, the reinforcing material may be secured to the first sheet and the second sheet by securing at least a periphery film layer to the first and second sheets. In FIG. 1, the reinforcing material is located towards the second sheet and between the fenestrations 112 because the tear lines extending from the notches would intersect the reinforcing material if the reinforcing material were located on all sides of the fenestrations. It is within the scope of the invention that the reinforcing material may formed in any shape or size depending on the arrangement of the fenestrations and the notches, and the type of surgery. If a particular surgery produces a large amount of fluid, then more reinforcing material may be used. For example the reinforcing material may be extended further onto the second sheet, thereby allowing the reinforcing material to absorb more fluid.

AII of the above described materials may be disposable and sterilizable. In particular the materials may be sterilizable by ethylene oxide treatment. It is within the scope of the invention, however, that any suitable sterilizing method may be used.

A method of using the surgical drape will now be described. If present, the adhesive material 116, located on an underside surface of the imperforate first sheet 110, is exposed. The adhesive may be exposed by removing a removable liner that protects the adhesive from exposure. The first sheet is placed against a patient's skin such that the fenestration 112 is positioned at the surgical site and is secured to the patient via the exposed adhesive. Surgical equipment, such as catheters, may be implemented within the surgical site. To remove the drape, a tear line is created by providing a tearing force at the notch 114. The tearing force is applied until the tear line 118 extends from the notch 114 to the fenestration along an imperforate portion of the first sheet. Additional tear lines may be formed by applying a tearing force at any other of the optional notches. These additional tear lines may be positioned to allow access to non-surgical sites, for example, a patient's head. After the tear line is completed, the drape may be removed from patient without disturbing the surgical equipment, such as catheters, that remain at the surgical site.

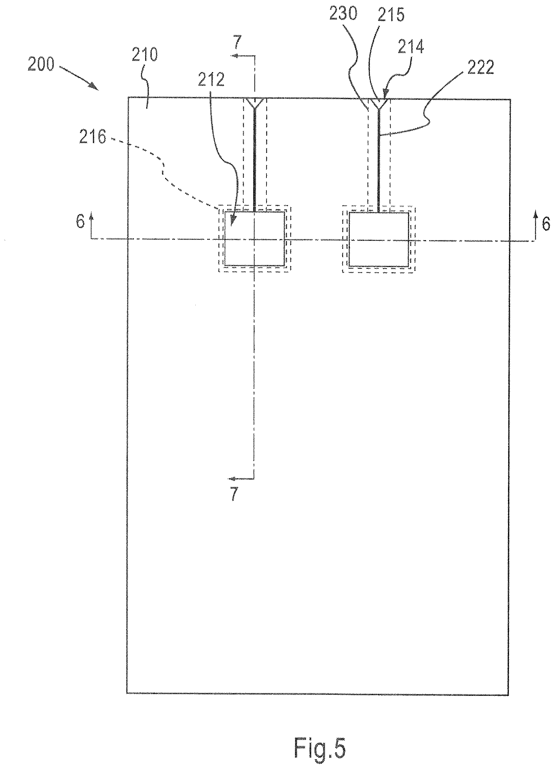

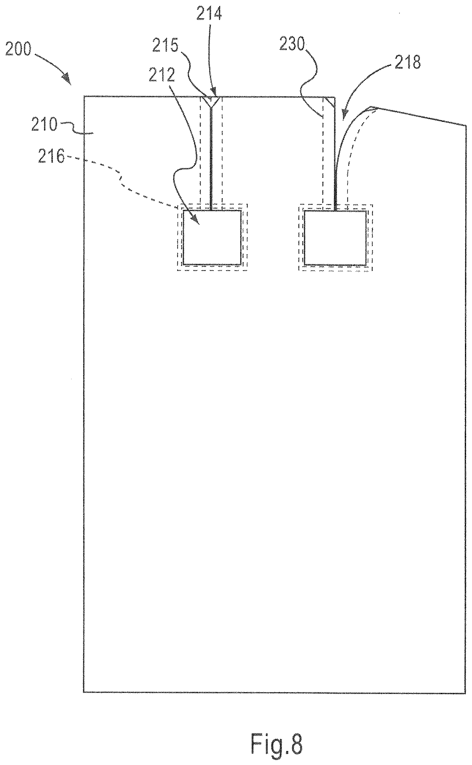

FIGS. 5 to 8 illustrate a second aspect of the surgical drape 200 for use during a surgical procedure. The surgical drape 200 comprises a first sheet 230, a second sheet 210, and an optional third sheet 220.

The second sheet 210 is a flexible sheet of material having a first and a second surface. As with the aspect of FIGs. 1-4, the second sheet is generally rectangular in a preferred aspect, but can be any suitable shape, provided it is large enough to cover a human body or a portion of the body adjacent the surgical site to assist in creating a sterile field at the surgical site. The second sheet may be imperforate as defined above at portions of the sheet that surround a cut 222, which is described below in more detail. In a preferred aspect, the second sheet 210 has at least one opening or fenestration 212. The fenestration may vary in position, shape, and number as described above with respect to the aspect of FIGs. 1-4. The optional third sheet 220 also has a first and second surface that generally overlaps and has the same dimensions as the second sheet 210. The optional third sheet is described in more detail below.

The aspect of FIGs. 5-8 differs from the aspect of FIGs. 1-4 in that at least one of the second sheet 210 and the optional third sheet 220 comprises a non-woven absorbent and/or reinforcing material. In particular, the entirety of at least one of sheets 210 and 220 may be formed entirely of the non-woven material described above. In the aspects of FIGS. 1-4, the non-woven material preferably is a separate layer from the main sheet and is disposed to avoid intersecting with the tear line formed in the main sheet and leading to the fenestration. Because the aspects of FIGs. 1-4 implemented a relatively thin sheet preferably comprising poly(ethylene-co-methacrylic acid) polymer, the sheet could be linearly torn after surgery while maintaining a barrier between the patient and surgery fluids during surgery. However, it is still desirable in particular surgeries that the entire sheet comprises a relatively thicker and more difficult to linearly tear non-woven material. This is particularly the case when a stronger drape is desirable or when it is desirable that the drape absorb fluids. A single sheet comprising a poly(ethylene-co-methacrylic acid) polymer may not adequately meet these needs but, as described above, provides a linear tear when torn making it easy to remove after surgery. A disadvantage of a non-woven fabric is that it may be difficult to tear, does not provide a linear tear when torn, or requires a tool to linearly tear/cut. However, a non-woven fabric may provide a stronger and/or absorbent quality that is desirable in certain circumstances. Thus, the aspect of FIGs. 5-8 solves this problem by providing a drape having a second sheet 210 and an optional third sheet 220, wherein at least one of the sheets is made of a non-woven fabric that is easily removed without tools while also providing a secure barrier between the patient and fluids before being torn. Thus, the invention achieves the benefits of a non-woven material while avoiding the disadvantages.

The aspects of FIGs. 5-8 achieve this goal by providing a cut 222 that extends from a periphery of the second sheet 210 and the third sheet 220 (if present) to the fenestration 212. Essentially, the sheets are pre-cut in a manner that facilitates removal of the drape from the patient. By already providing a cut in the sheets before the surgery, it is not necessary to cut or tear the non-woven sheets after the surgery. However, having a pre-cut in the sheets does not maintain a barrier between the surgical fluids and the patient. To retain the pre-cut feature and the barrier in a single drape, a first sheet 230 having an imperforate portion is affixed to the first or underside surface of the second sheet. The first sheet 230, and more particularly, the imperforate portion of the first sheet, preferably extends along the length of and overlaps the cut 222. As illustrated in FIGs. 5, 7, and 8, the first sheet may also extend beyond the cut to a periphery of the second sheet. Imperforate has the same meaning described above with respect to the imperforate portion of the first sheet of FIGs. 1-4. As best seen in FIG. 5, the first sheet 230 is affixed on opposing sides of the cut 222. The first sheet essentially acts as a barrier member that that stops fluid from passing through the cut and onto the patient.

The first sheet 230 of FIGs. 5-8 is analogous to the first sheet 110 of FIGs. 1-4 in that both sheets are linearly tearable at least along a portion of the sheet extending from a notch to a fenestration, and therefore, the first sheet 230 may comprise the same material described above with respect to the first sheet 110 of the aspects of FIGs 1-4.

As with the aspects of FIGs 1-4, the drape 200 of FIGs. 5-8 further includes at least one notch 214 located at a periphery of the first sheet and a periphery of the second sheet to facilitate tearing of first sheet once it is desirable to remove the drape. The notch 214 is aligned or oriented with the cut 222 such that when a tearing motion is applied to the notch, a linear tear line 218 is propagated along the length of the first sheet 230, and in particular, along the imperforate portion of the first sheet. The term notch has the same meaning as defined above. In a preferred aspect the notch is positioned such that the notch directly opposes the fenestration. As illustrated in FIGs. 5, 7, and 8, when the first sheet extends to the periphery of the second sheet, a second notch 215 may be disposed at the first sheet periphery, which is distinct from the notch 214 disposed at a periphery of the second sheet. The aspect of the drape having two notches allows the practitioner to locate the notch in the film while also allowing the practitioner to locate the film.

It is within the scope of the invention that any number of cuts along with corresponding sheets and notches may be used and in any combination. For example, as shown in FIG. 5, each fenestration has a first cut, a first sheet, a first notch, and a second notch disposed in a vertical direction. Any arrangement of cuts, sheets, and notches on the drape is within the scope of the invention. For example, each fenestration could have a corresponding single cut, tearable sheet, and notches located in a horizontal direction, each fenestration could have a corresponding single cut, tearable sheet, and notches located in a vertical direction, one fenestration could have a corresponding single cut, tearable sheet, and notches in a horizontal direction while another fenestration has a corresponding single cut, tearable sheet, and notches located in a vertical direction, one fenestration could have both a cut, tearable sheet, and notches in a horizontal direction and a cut, tearable sheet, and notches in a horizontal direction, or one fenestration could have at least one cut, tearable sheet, and notches associated with it while another fenestration has no cut, tearable sheet, and notches associated with it. The first sheet may optionally comprise additional cuts, sheets, and notches that do not line up with the fenestrations. For example, a cut, tearable sheet, and notches may be disposed at a periphery of the second sheet such that a tear line originating at the notch will expose the head of a patient without intersecting with a fenestration.

As with the aspects of FIGs. 1-4, in preferred aspects of FIGs. 5-8, the notch may be triangular in shape. As shown in FIG. 5, the triangular notch is oriented such that a point of the notch points directly towards the fenestration 212. Furthermore, in an aspect, the notch 214 formed in the second sheet may be triangular, while the second notch 215 formed in the first sheet may be a slit.

Because the composition of at least a portion of the first sheet allows for linear tear lines (i.e., the portion extending from the notch to the fenestration), when the first sheet and the second sheet (and third sheet if present) is spread apart at the notch, a tear line 218 forms at the notch and continuously extends perpendicular to the base of the triangle notch or, generally, the periphery of the first sheet on which the notch is located, in a direction of the fenestration 212, until it reaches the fenestration. In another aspect, when the first sheet is spread apart at the second notch 215, the tear line 218 begins to form before the second or third sheet is spread apart at the notch 214. As the tear propagates, the barrier provided by the first sheet is broken and the cut 222 is exposed. After the tear line is completely formed between the notch and the fenestration, the cut is fully exposed, and the surgeon can easily remove the drape without disturbing any surgical equipment present inside of the fenestration.

Because at least a portion of the first sheet is tearable in a linear manner, no perforations or score lines need to be formed through the first sheet or the sheets and no additional equipment, such as scissors is necessary to gain access to the fenestration. This arrangement provides several advantages. First, a complete barrier is provided between the patient and the fluids, thereby preventing fluid from leaking through the drape. Second, there is little or no risk of accidental tearing when the drape is manipulated, such as when positioning the drape over the patient before a surgery. Third, the drape is structurally secure at all points along the drape. However, it is within the scope of the invention that perforations may be implemented at parts of the drape distinct from the notch and tear line described above.

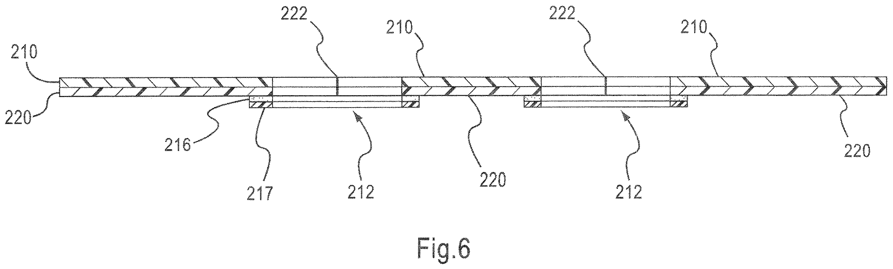

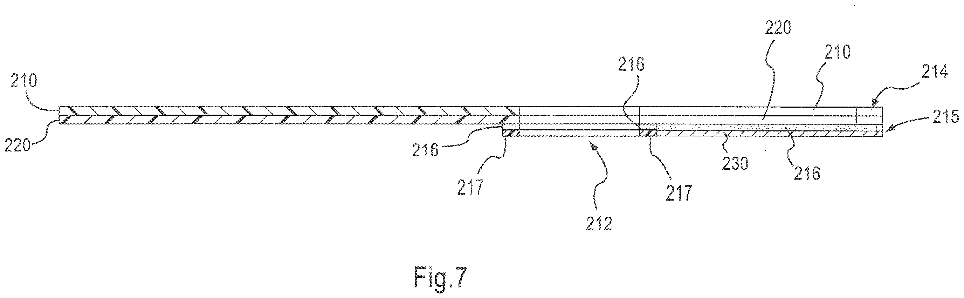

As best seen in FIG. 7, the first sheet 230 optionally further comprises an adhesive 216 disposed on an underside surface of the first sheet, i.e., the side that contacts the patient when in use. The adhesive is preferably disposed along the periphery of the cut to secure the first sheet around and overlapping the cut. In a preferred aspect the adhesive may be an acrylic adhesive. It is within the scope of the invention however that any suitable adhesive may be used, for example, synthetic rubber. As seen in FIGs. 6 and 7, the adhesive may be applied to the underside surface of second sheet. The adhesive and the first sheet may be applied directly to the second sheet, e.g. with hot melt equipment. Generally, enough adhesive should be used to securely attach the first sheet to the second sheet so that the first sheet is not removable even with significant force. Strongly securing the first sheet to the second sheet avoids the chance of the first sheet being accidently removed.

As discussed above, the drape 200 optionally comprises a third sheet 220. While the third sheet is not required, it is often desirable to have a third sheet to add stability or other functionality to the drape. The third sheet may have the same size and shape of the second sheet and be secured to second sheet. Depending on the materials chosen for the second and third sheets, the sheets can be secured along a periphery using the same adhesive techniques described above. In an aspect of the invention, the second sheet may comprise the same material as the first sheet, e.g. a polymer preferably a methacrylate polymer such as poly(ethylene-co-methacrylic acid), while the third sheet may comprise the non-woven fabric discussed above. This arrangement provides a non-absorbent liquid repellant layer closest to the patient and an absorbent and/or reinforcing layer on top. Thus, the top layer may be used to absorb fluids and/or provide stability to the drape. In another aspect both of the second and third sheets are made of the above-described non-woven materials. When both sheets are non-woven, both sheets may provide absorbency and/or support. Because of the cut feature, there is no limitation on the thickness or strength of material that may be used for the second and third sheets. Thus, it is within the scope of the invention that any desirable material having any desirable thickness may be implemented in the invention. The particular selection of material is dependent on the type of surgery being performed and in particular, whether it is desirable to collect or repel the fluids produced by the surgery, and how much fluid is produced during the surgery.

When a third sheet is implemented, the fenestration must also pass through the third sheet to allow access to the surgical site. For the third sheet to be removed, the same cut feature described above with respect to the second sheet may be included in the third sheet. Thus, a second cut may extend from a periphery of the third sheet to the fenestration. The second cut of the third sheet preferably overlaps the cut of the second sheet and the notch 214 is also disposed at the periphery of the third sheet. With this arrangement, when the tearing force is applied to the notch 214 or second notch 215, the first sheet will tear as described above and allow the removal of both the second and third sheets at the same time. Furthermore, it should be evident while the aspect of FIGs. 5-8 shows three sheets, it is within the scope of the invention that any number of additional sheets may be added. As long as a cut feature is implemented in each additional sheet, the drape will be easy to remove after the first sheet on the bottom of the drape is torn.

Other aspects of FIGs. 5-8 are the same as disclosed above with respect to the aspect of FIGs. 1-4. For example, the manner in which the fenestration is secured around the surgical site, including the adhesive and double-sided tape on the bottom sheet or a single sided tape on the top sheet, is the same as described above. For example, as shown in FIGs. 6 and 7, an adhesive 216 may be applied to the first surface of the second sheet 220 and a removable liner 217 may be applied to the other side of the adhesive. Furthermore, additional sheets may be secured to the periphery of second and third sheets to extend the size of the drape depending on the particular surgery. For example, an absorbent or repellent sheet may be adhered to the periphery of the sides of the second and third sheets to cover additional equipment or people in the operating room.

A method of using the surgical drape 200 will now be described. The application of the drape to the patient follows the same steps as described above with the respect to the surgical drape 100. The method differs when removing the drape. To remove the drape, a tear line in the first sheet 230 is created by providing a tearing force at the notch 214 or the second notch 215. The tearing force is applied until the tear line 218 extends along the cut 222 from the notch 214 or second notch 215 to the fenestration. Additional tear lines may be formed by applying a tearing force at any other of the optional notches. These additional tear lines may be positioned to allow access to non-surgical sites, for example, a patient's head. After the tear line is completed, the drape may be removed from patient without disturbing the surgical equipment, such as catheters, that remain at the surgical site.

The invention has been described herein with reference to various specific and preferred materials, aspects and techniques. Therefore, the invention should not be limited by the above description, and to ascertain the full scope of the invention, the following aspects should be referenced.

## Claims

1. A surgical drape comprising:
a tearable first sheet (110), the first sheet (110) comprising:
at least one notch (114) disposed at a periphery of the first sheet (110);
at least one fenestration (112), and wherein the at least one fenestration (112) provides access to a surgical site; and
an imperforate portion extending from the at least one notch (114) to the at least one fenestration (112), and
wherein tearing the first sheet (110) via the at least one notch (114) creates a linear tear line extending along the imperforate portion from the at least one notch (114) to the at least one fenestration (112), and **characterized in that**,
the surgical drape comprises a reinforcing material (130) disposed on an upper surface of the first sheet (110) wherein the reinforcing material (130) is absorbent, and wherein, the linear tear line does not intersect the reinforcing material (130).

2. The surgical drape of claim 1, wherein the at least one notch (114) comprises a first notch disposed in a horizontal direction from the at least one fenestration (112) and a second notch disposed in a vertical direction from the at least one fenestration (112).

3. The surgical drape of claim 1, wherein the at least one notch (114) comprises a triangle shape having a point pointing towards the at least one fenestration (112).

4. The surgical drape of claim 1, wherein the imperforate portion comprises poly(ethylene-co-methacrylic acid) polymers.

5. The surgical drape of claim 4, wherein the imperforate portion comprises three layers laminated together, wherein each of the three layers comprises poly(ethylene-co-methacrylic acid) polymers.

6. The surgical drape of claim 1, further comprising an adhesive disposed proximate to the at least one fenestration (112) and on an underside surface of the first sheet (110), wherein the adhesive comprises a tearable carrier material, and wherein the carrier material is a paper tissue.

7. The surgical drape of claim 6, wherein the adhesive comprises a removable liner (117).

8. The surgical drape of claim 1, further comprising a second sheet (120) attached to an upper surface of the first sheet (110).

9. The surgical drape of claim 8, wherein the second sheet (120) is attached to the first sheet (110) via an adhesive.

10. The surgical drape of claim 8, wherein the reinforcing material (130) is disposed on an upper surface of the second sheet (120).

11. The surgical drape of claim 10, wherein the reinforcing material (130) is disposed proximate to the at least one fenestration (112).

12. The surgical drape of claim 1, further comprising:
a second sheet (210) having a first and a second surface, wherein the second sheet (210) comprises the at least one fenestration (212), wherein the at least one fenestration (212) provides access to a surgical site, wherein a cut (222) extends from a periphery of the second sheet (210) to the at least one fenestration (212) in the second sheet (210), wherein the first sheet (230) is affixed to the first surface of the second sheet (210) and extends along a length of and overlapping the cut (222), and wherein the at least one notch (214) is disposed at the periphery of the second sheet (210) and is aligned with the cut (222).

13. The surgical drape of claim 12, wherein the first sheet is affixed to a portion of the second sheet (210) on opposing sides of the cut (222).

14. The surgical drape of claim 12, further comprising a third sheet (220) affixed to the second surface of the second sheet (210), wherein the at least one fenestration (212) passes through the second (210) and third sheet (220).

15. The surgical drape of claim 14, wherein a second cut extends from a periphery of the third sheet (220) to the at least one fenestration (212), wherein the second cut of the third sheet (220) overlaps the cut (222) of the second sheet (210), and wherein the at least one notch (214) is disposed at the periphery of the third sheet (220).

16. The surgical drape of claim 15, wherein the second (210) and third sheets (220) comprise an absorbent material.

17. The surgical drape of claim 14, where the second sheet (210) comprises a fluid repelling material and the third sheet (220) comprises an absorbent material.

18. The surgical drape of claim 14, wherein the second sheet (210) and the third sheet (220) comprise a non-woven fabric.

19. The surgical drape of claim 14, wherein the second sheet (210) comprises poly(ethylene-co-methacrylic acid) polymers and the third sheet (220) comprises a non-woven fabric.

20. The surgical drape of claim 12, wherein the first sheet (230) is affixed to the second sheet (210) via an adhesive.

21. The surgical drape of claim 14, further comprising an adhesive disposed proximate to the at least one fenestration (212) and on the first surface of the second sheet (210) for securing the at least one fenestration (212) around the surgical site.

22. The surgical drape of claim 12, wherein the imperforate portion of the first sheet (230) extends to the periphery of the second sheet (210), and wherein the least one notch (214) comprises a first notch (215) disposed at the periphery of the first sheet (230) and a second notch (214) disposed at the periphery of the second sheet (210).

23. A method of removing the surgical drape of claim 1, the method comprising tearing the first sheet (110) by applying a tearing force at the at least one notch (114), and removing the surgical drape from a surgical site.

## Patentansprüche

1. Operationsfeld, das Folgendes umfasst:
eine abreißbare erste Bahn (110), wobei die erste Bahn (110) Folgendes umfasst:
mindestens eine Kerbe (114), die an einem Rand der ersten Bahn (110) angeordnet ist,
mindestens eine Fensterung (112) und wobei die mindestens eine Fensterung (112) Zugang zu einer Operationsstelle gewährt, und
einen nicht perforierten Abschnitt, der sich von der mindestens einen Kerbe (114) bis zu der mindestens einen Fensterung (112) erstreckt, und
wobei ein Abreißen der ersten Bahn (110) über die mindestens eine Kerbe (114) eine lineare Aufreißlinie erzeugt, die sich entlang des nicht perforierten Abschnitts von der mindestens einen Kerbe (114) bis zu der mindestens einen Fensterung (112) erstreckt, und **dadurch gekennzeichnet, dass**
das Operationsfeld ein Verstärkungsmaterial (130) umfasst, das auf einer oberen Fläche der ersten Bahn (110) angeordnet ist, wobei das Verstärkungsmaterial (130) absorbierend ist und wobei die lineare Aufreißlinie das Verstärkungsmaterial (130) nicht schneidet.

2. Operationsfeld nach Anspruch 1, wobei die mindestens eine Kerbe (114) eine erste Kerbe, die in einer horizontalen Richtung von der mindestens einen Fensterung (112) angeordnet ist, und eine zweite Kerbe, die in einer vertikalen Richtung von der mindestens einen Fensterung (112) angeordnet ist, umfasst.

3. Operationsfeld nach Anspruch 1, wobei die mindestens eine Kerbe (114) eine dreieckige Form umfasst, die eine Spitze aufweist, die hin zu der mindestens einen Fensterung (112) zeigt.

4. Operationsfeld nach Anspruch 1, wobei der nicht perforierte Abschnitt Poly(ethylen-co-methacrylsäure)-Polymere umfasst.

5. Operationsfeld nach Anspruch 4, wobei der nicht perforierte Abschnitt drei Lagen umfasst, die miteinander laminiert sind, wobei jede der drei Lagen Poly(ethylen-co-methacrylsäure)-Polymere umfasst.

6. Operationsfeld nach Anspruch 1, das ferner einen Klebstoff umfasst, der nahe der mindestens einen Fensterung (112) und auf einer Unterseitenfläche der ersten Bahn (110) angeordnet ist, wobei der Klebstoff ein abreißbares Trägermaterial umfasst und wobei das Trägermaterial ein Papiertuch ist.

7. Operationsfeld nach Anspruch 6, wobei der Klebstoff eine entfernbare Auskleidung umfasst.

8. Operationsfeld nach Anspruch 1, das ferner eine zweite Bahn (120) umfasst, die an einer oberen Fläche der ersten Bahn (110) befestigt ist.

9. Operationsfeld nach Anspruch 8, wobei die zweite Bahn (120) über einen Klebstoff an der ersten Bahn (110) befestigt ist.

10. Operationsfeld nach Anspruch 8, wobei das Verstärkungsmaterial (130) auf einer oberen Fläche der zweiten Bahn (120) angeordnet ist.

11. Operationsfeld nach Anspruch 10, wobei das Verstärkungsmaterial (130) nahe der mindestens einen Fensterung (112) angeordnet ist.

12. Operationsfeld nach Anspruch 1, das ferner Folgendes umfasst:
eine zweite Bahn (210), die eine erste und eine zweite Fläche aufweist, wobei die zweite Bahn (210) die mindestens eine Fensterung (212) umfasst, wobei die mindestens eine Fensterung (212) Zugang zu einer Operationsstelle gewährt, wobei sich ein Schnitt (222) von einem Rand der zweiten Bahn (210) bis zu der mindestens einen Fensterung (212) in der zweiten Bahn (210) erstreckt, wobei die erste Bahn (230) an der ersten Fläche der zweiten Bahn (210) befestigt ist und sich entlang einer Länge des Schnittes (222) erstreckt und denselben überlappt und wobei die mindestens eine Kerbe (214) an einem Rand der zweiten Bahn (210) angeordnet ist und mit dem Schnitt (222) ausgerichtet ist.

13. Operationsfeld nach Anspruch 12, wobei die erste Bahn auf entgegengesetzten Seiten des Schnittes (222) an einem Abschnitt der zweiten Bahn (210) befestigt ist.

14. Operationsfeld nach Anspruch 12, das ferner eine dritte Bahn (220) umfasst, die an der zweiten Fläche der zweiten Bahn (210) befestigt ist, wobei die mindestens eine Fensterung (212) durch die zweite (210) und die dritte Bahn (220) hindurchgeht.

15. Operationsfeld nach Anspruch 14, wobei sich ein zweiter Schnitt von einem Rand der dritten Bahn (220) bis zu der mindestens einen Fensterung (212) erstreckt, wobei der zweite Schnitt der dritten Bahn (220) den Schnitt (222) der zweiten Bahn (210) überlappt und wobei die mindestens eine Kerbe (214) an einem Rand der dritten Bahn (220) angeordnet ist.

16. Operationsfeld nach Anspruch 15, wobei die zweite (210) und die dritte Bahn (220) ein absorbierendes Material umfasst.

17. Operationsfeld nach Anspruch 14, wobei die zweite Bahn (210) ein fluidabweisendes Material umfasst und die dritte Bahn (220) ein absorbierendes Material umfasst.

18. Operationsfeld nach Anspruch 14, wobei die zweite (210) und die dritte Bahn (220) einen Vliesstoff umfassen.

19. Operationsfeld nach Anspruch 14, wobei die zweite Bahn (210) Poly(ethylen-co-methacrylsäure)-Polymere umfasst und die dritte Bahn (220) einen Vliesstoff umfasst.

20. Operationsfeld nach Anspruch 12, wobei die erste Bahn (230) über einen Klebstoff an der zweiten Bahn (210) befestigt ist.

21. Operationsfeld nach Anspruch 14, das ferner einen Klebstoff umfasst, der nahe der mindestens einen Fensterung (212) und auf der ersten Fläche der zweiten Bahn (210) angeordnet ist, zum Befestigen der mindestens einen Fensterung (212) um die Operationsstelle.

22. Operationsfeld nach Anspruch 12, wobei sich der nicht perforierte Abschnitt der ersten Bahn (230) bis zu dem Rand der zweiten Bahn (210) erstreckt und wobei die mindestens eine Kerbe (214) eine erste Kerbe (215), die an dem Rand der ersten Bahn (230) angeordnet ist, und eine zweite Kerbe (214), die an dem Rand der zweiten Bahn (210) angeordnet ist, umfasst.

23. Verfahren zum Entfernen des Operationsfelds nach Anspruch 1, wobei das Verfahren das Abreißen der ersten Bahn (110) durch Anwenden einer Abreißkraft an der mindestens einen Kerbe (114) und das Entfernen des Operationsfelds von einer Operationsstelle umfasst.

## Revendications

1. Champ opératoire, comprenant :
une première feuille déchirable (110), la première feuille (110) comprenant :
au moins une encoche (114) dispose au niveau d'une périphérie de la première feuille (110) ;
au moins une fenêtre (112), et dans lequel la au moins une fenêtre (112) établit un accès à un site opératoire ; et
une partie non perforée s'étendant de la au moins une encoche (14) vers la au moins une fenêtre (112) ; et
dans lequel la déchirure de la première feuille (110) à travers la au moins une encoche (114) établit une ligne de déchirure linéaire le long de la partie non perforée, de la au moins une encoche (114) vers la au moins une fenêtre (112), et **caractérisé en ce que** :
le champ opératoire comprend un matériau de renforcement (130) disposé sur une surface supérieure de la première feuille (110), dans lequel le matériau de renforcement (130) est absorbant, et dans lequel la ligne de déchirure linéaire ne coupe pas le matériau de renforcement (130).

2. Champ opératoire selon la revendication 1, dans lequel la au moins une encoche (114) comprend une première encoche disposée dans une direction horizontale par rapport à la au moins une fenêtre (112), et une deuxième encoche disposée dans une direction verticale par rapport à la au moins une fenêtre (112).

3. Champ opératoire selon la revendication 1, dans lequel la au moins une encoche (114) a une forme triangulaire comportant une pointe orientée vers la au moins une fenêtre (112).

4. Champ opératoire selon la revendication 1, dans lequel la partie non perforée comprend des polymères d'acide poly(éthylène-co-méthacrylique).

5. Champ opératoire selon la revendication 4, dans lequel la partie non perforée comprend trois couches starifiées ensemble, dans lequel chacune de trois couches comprend des polymères d'acide poly(éthylène-co-méthacrylique).

6. Champ opératoire selon la revendication 1, comprenant en outre un adhésif disposé à proximité de la au moins une fenêtre (112) et sur une surface inférieure de la première feuille (110), dans lequel l'adhésif comprend un matériau de support déchirable, et dans lequel le matériau de support est un mouchoir en papier.

7. Champ opératoire selon la revendication 6, dans lequel l'adhésif comprend une doublure amovible.

8. Champ opératoire selon la revendication 1, comprenant en outre une deuxième feuille (120) fixée sur une surface supérieure de la première feuille (110).

9. Champ opératoire selon la revendication 8, dans lequel la deuxième feuille (120) est fixée sur la première feuille (110) par l'intermédiaire d'un adhésif.

10. Champ opératoire selon la revendication 8, dans lequel le matériau de renforcement (130) est disposé sur une surface supérieure de la deuxième feuille (120).

11. Champ opératoire selon la revendication 10, dans lequel le matériau de renforcement (130) est disposé à proximité de la au moins une fenêtre (112).

12. Champ chirurgical selon la revendication 1, comprenant en outre :
une deuxième feuille (210) comportant une première et une deuxième surface, dans lequel la deuxième feuille (210) comprend la au moins une fenêtre (212), dans lequel la au moins une fenêtre (212) établit un accès à un site opératoire, dans lequel une découpe (222) s'étend de la périphérie de la deuxième feuille (212) vers la au moins une fenêtre (212) dans la deuxième feuille (210), dans lequel la première feuille (230) est fixée sur la première surface de la deuxième feuille (210) et s'étend le long d'une longueur de la découpe (222) et chevauche celle-ci, et dans lequel la au moins une encoche (24) et disposée au niveau de la périphérie de la deuxième feuille (210) et est alignée avec la découpe (222).

13. Champ opératoire selon la revendication 12, dans lequel la première partie est fixée sur une partie de la deuxième feuille (210) sur des côtés opposés de la découpe (222).

14. Champ opératoire selon la revendication 2, comprenant en outre une troisième feuille (220) fixée sur la deuxième surface de la deuxième feuille (210), dans lequel la au moins une fenêtre (212) passe à travers les deuxième (210) et troisième feuilles (220).

15. Champ opératoire selon la revendication 14, dans lequel une deuxième découpe s'étend d'une périphérie de la troisième feuille (220) vers la au moins une fenêtre (212), dans lequel la deuxième découpe de la troisième feuille (220) chevauche la découpe (222) de la deuxième feuille (210), et dans lequel la au moins une encoche (214) est disposée au niveau de la périphérie de la troisième feuille (220).

16. Champ opératoire selon la revendication 15, dans lequel les deuxième (210) et troisième (220) feuilles comprennent un matériau absorbant.

17. Champ opératoire selon la revendication 14, dans lequel la deuxième feuille (210) comprend un matériau repoussant les fluides et la troisième feuille (220) comprend un matériau absorbant.

18. Champ opératoire selon la revendication 14, dans lequel la deuxième feuille (210) et la troisième feuille (220) comprennent un tissu non tissé.

19. Champ opératoire selon la revendication 14, dans lequel la deuxième feuille (210) comprend des polymères d'acide poly(éthylène-co-méthacrylique) et la troisième feuille (220) comprend un tissu non tissé.

20. Champ opératoire selon la revendication 12, dans lequel la première feuille (230) est fixée sur la deuxième feuille (210) par l'intermédiaire d'un adhésif.

21. Champ opératoire selon la revendication 14, comprenant en outre un adhésif disposé à proximité de la au moins une fenêtre (212) et sur la première surface de la deuxième feuille (210) pour fixer la au moins une fenêtre (212) autour du site chirurgical.

22. Champ opératoire selon la revendication 12, dans lequel la partie non perforée de la première feuille (230) s'étend vers la périphérie de la deuxième feuille (210), et dans lequel la au moins une encoche (214) comprend une première encoche (215) disposée au niveau de la périphérie de la première feuille (230), et une deuxième encoche (214) disposée au niveau de la périphérie de la deuxième feuille (210).

23. Procédé de retrait du champ opératoire selon la revendication 1, le procédé comprenant les étapes de déchirure de la première feuille (110) en appliquant une force de déchirure sur la au moins une encoche (114) et de retrait du champ opératoire d'un site chirurgical.
